# EUROPÄISCHE PATENTSCHRIFT

(11) **EP 0 398 064 B1**
(45) Veröffentlichungstag und Bekanntmachung des Hinweises auf die Patenterteilung: **09.02.1994**
(21) Anmeldenummer: 90108189.3
(22) Anmeldetag: 28.04.1990
(51) Int. Cl.: A61F 2/36, A61F 2/30

(54) **Schaft einer Hüftgelenkprothese und Verfahren zu dessen Herstellung**
Stem of a hip joint prosthesis and method for its production
Tige de prothèse de hanche et procédé pour sa fabrication

(30) Priorität: 17.05.1989 DE 3915983; 13.06.1989 DE 3919192; 28.07.1989 DE 3924990
(43) Veröffentlichungstag der Anmeldung: 22.11.1990
(73) Patentinhaber: MAN Ceramics GmbH, 94453 Deggendorf (DE)
(72) Erfinder: Wenner, Ulrich, Dipl.-Ing., D-3110 Uelzen 1 (DE)

(56) Entgegenhaltungen:
- DE-A- 2 512 152
- DE-A- 2 753 568
- DE-A- 3 243 861
- DE-A-31 376 52
- DE-A-39 095 45

## Beschreibung

Die Erfindung bezieht sich auf einen Schaft sowie auf ein Verfahren zur Herstellung eines Schaftes für eine Hüftgelenkprothese aus faserverstärktem Kunststoff, wobei die Fasern in Längsrichtung des Schaftes verlaufen und zumindest teilweise von einem Schaftende zum anderen Schaftende durchgehen.

Aus der DE 27 53 568 C3 ist ein Schaft dieser Art bekannt, bei dem die Fasern in Längsrichtung des Schaftes verlaufen, wobei lediglich die an der Oberfläche befindlichen Fasern von einem Ende bis zum anderen Ende des Schaftes reichen. Dieses hat zur Folge, daß Kräfte, die auf das obere Ende des Schaftes auferlegt werden, nur von einem Teil der Fasern an einen Krümmungsbereich des Schaftes eingeleitet werden können, zumal die inneren Fasern dieses Schaftendes zum Teil den Krümmungsbereich nicht erreichen.

Gemäß einer aus der DE 32 43 861 bekannten Ausführung besteht der Schaft ausschließlich aus unidirektionalen Fasern, die jedoch aufgrund der zu den Schaftenden stark abnehmenden Querschnittsflächen nur zu einem geringen und örtlich undefinierten Anteil von einem Schaftende bis zum anderen Schaftende reichen.

Der Erfindung liegt die Aufgabe zugrunde, einen Schaft der eingangs genannten Art zu schaffen, bei dem Kräfte optimal im Schaft weitergeleitet werden können.

Die Aufgabe wird erfindungsgemäß mit den Merkmalen des Anspruchs 1 gelöst.

Hierbei bildet ein geometrisch unregelmäßiger Kern einen inneren Verdrängerkörper, um den die für die Steifigkeit des Schaftes verantwortlichen Fasern von einem Schaftende bis zum anderen Schaftende durchgehend gelegt sind, so daß die Fasern parallel zueinander in Längsrichtung des Schaftes geführt sind. Hierdurch werden sämtliche, am oberen, die Kräfte aufnehmenden Ende des Schaftes endenden Fasern an der Krafteinleitung in andere Bereiche des Schaftes beteiligt, wodurch eine optimale Nutzung der Verstärkungseigenschaften der Fasern gewährleistet ist.

Der Kraftschluß bleibt in der Ummantelung, d. h. an der Schaftoberfläche bzw. in der Umgebung des umliegenden Knochenmaterials im einplantierten Zustand. Damit werden die Kräfte über die Fasern direkt an die Knochenanwachszone oder gegebenenfalls an ein dazwischenliegendes Klebmaterial eingeleitet. Es gibt keine Zwischenbauteile, die sich aufgrund der Belastungen lösen könnten.

Die Fasern werden zumindest an einem Schaftende den vollen Querschnitt ausfüllen, während sie im übrigen Bereich den Kern umgeben. Wesentlich ist dabei, daß die Querschnittsfläche der Ummantelung am Schaftende und im Kernbereich stets dieselbe ist, das heißt, im Querschnitt enthält die Ummantelung über die gesamte Länge des Schaftes die gleiche Anzahl von Fasern.

Die Erfindung erstreckt sich auf ein Verfahren zur Herstellung eines Schaftes gemäß Anspruch 1. Das erfindungsgemäße Verfahren ist durch die Merkmale des Anspruchs 3 gekennzeichnet.

Bei dem bekannten Verfahren nach der DE 27 53 568 C3 wird mit den Fasern ein Ring gewickelt, dessen Kontur abschnittsweise Konturenteilen des herzustellenden Schaftes entspricht. Diese Abschnitte werden nach dem Aushärten des Ringes vom Ring abgetrennt und miteinander zur Bildung des Schaftes zusammengeklebt. Mit diesem Verfahren ist es zwar möglich, den geometrisch unregelmäßigen Schaft zumindest an der Oberfläche mit durchgehenden Längsfasern herzustellen, jedoch ist dieses für einen Schaft mit annähernd rundem Querschnitt mit großen Schwierigkeiten verbunden und überhaupt für die weiteren Fasern unmöglich.

Bei dem erfindungsgemäßen Verfahren ist es dagegen durch die Herstellung von zwei oder mehr über die Schaftlänge verlaufenden Schalen möglich, alle in einem Schaftende auftretenden Fasern durchgehend bis zum anderen Ende durchzuziehen.

Bei zwei Schalen wird ein Längsschnitt des Schaftes vorgesehen, dessen Schnittfläche senkrecht zur Ebene verläuft, in der die Schaftkrümmung liegt. Auf diese Weise ergeben sich zwei Teilkomponenten, die eine wickelbare Krümmung, nämlich die Schaftkrümmung, aufweisen. Die Längsrichtung der Schalen entsprechen somit der Längsrichtung des Schaftes. Die längsgerichteten Fasern bündeln sich in den beiden Schaftbereichen, während sie im Mittelbereich, sich auffächernd, die schalenförmige Struktur bilden. Werden die Schalen miteinander verbunden, so ergibt sich ein Hohlkörper, dessen Wandung aus parallel zur Wandung verlaufenden, unidirektionalen Fasern gebildet ist. Damit ist eine optimale Krafteinleitung vom oberen Ende eines Schaftes auf den aufgeweiteten Mittelbereich möglich. Radialen Kräften wird mit dem den Hohlraum lückenlos ausfüllenden Kern entgegengewirkt.

Der erfindungsgemäß hergestellte Schaft ist kompakt, und dessen Außenkontur kann genau an optimale Formgebungen angepaßt werden. Insbesondere konische Endbereiche lassen sich formgenau mit durchlaufenden Fasern herstellen.

Gemäß einer weiteren Ausgestaltung der Erfindung werden die Schalen und der Kern als Prepreg-Teilkomponenten, d. h. nicht vorgehärtet, hergestellt und erst nach dem Zusammenfügen unter Wärmeeinwirkung gehärtet und ausgehärtet. Das hat den Vorteil, daß die Fasermatrix-Struktur an den Fügestellen sich homogen fortsetzt und so keine Klebstellen oder Struktursprungstellen im Schaft vorkommen. Die für die Steifigkeit verantwortlichen Schalen bilden damit zusammen einen homogenen Körper.

Für den Aushärtungsvorgang werden die Teilkomponenten vorzugsweise in eine Form gebracht und während des Aushärtens unter Druck gehalten. Die Schnittfläche der Form ist vorzugsweise senkrecht zur Schnittfläche der Halbschalen gerichtet. Vorzugsweise wird ein geringer Druck verwendet, der gerade ausreicht, um einen guten flächendeckenden Kontakt zwischen den Teilkomponenten zu gewährleisten. Damit wird kein Matrixmaterial herausgequetscht.

Es ist selbstverständlich möglich, den Schaft anstatt aus zwei Halbschalen aus drei oder mehr Schalen zusammenzustellen, wenn dieses aus fertigungstechnischen Gründen und wegen der Schaftform, insbesondere zum Wickeln der Schalen, günstiger erscheint. Im allgemeinen wird man mit zwei Halbschalen auskommen und dieser Aufteilung den Vorzug geben, insbesondere wenn für verschiedene Schaftgrößen jeweils gesonderte Wickeleinrichtungen vorgesehen werden müssen.

Für die Formgebung einer Schale wird vorzugsweise eine Negativform verwendet, die je nach der Schalenkontur eine konvexe Oberfläche oder eine konkave Oberfläche hat, auf die bzw. in die die imprägnierten Fasern der Länge nach gewickelt oder mit Fadenspannung eingelegt werden. In der Längsrichtung der Schalen (diese Richtung entspricht der Längsrichtung des fertigen Schaftes) ist die Negativform jeweils konvex, so daß ein Wickelverfahren überhaupt möglich ist. Die Querrichtung dazu kann dagegen konvex oder konkav sein. Zur Überwindung dieser Stellen werden Stützformen verwendet, die ein Abrutschen der Fasern verhindern. An den Enden der Negativform sind jeweils Hilfsstifte vorgesehen, um die die Faser für den Rücklauf umgelenkt wird. Auf diese Weise ist ein mechanisches Wickelverfahren möglich, bei dem die Fasern in Pendelbewegung hin- und hergeführt und an den Stiften umgelenkt werden.

Die Schaftenden werden im allgemeinen voll aus den gebündelten, unidirektionalen Fasern, d. h. ohne einen zentrischen Hohlraum bestehen, insbesondere dann, wenn die beiden Schaftenden einen gleichen Querschnitt haben. Die Fasern werden im Zwischenbereich fächerartig in einer oder mehr Lagen nebeneinanderliegend eine Schalenform bilden. Auf diese Weise verlaufen sämtliche Fasern von einem Schaftende zum anderen Schaftende, wobei sie im Bereich dazwischen sich homogen auf den Umfang des zylinderartigen Schalenbereiches verteilen. Dieser Bereich liegt ungefähr an der Knickstelle des Schaftes, worüber die Krafteinleitung der Fasern auf die umgebene Knochenwandung erfolgt. Wenn hier unterschiedliche Beanspruchungen berücksichtigt werden müssen, dann kann die Faserverteilung im Schalenbereich entsprechend inhomogen gewählt werden. Nach dem erfindungsgemäßen Verfahren ist es möglich, konische Schäfte zu bilden, bei denen sämtliche Fasern den Konus vom kleinen bis zum größeren Querschnitt durchlaufen. Im größeren Querschnittbereich wird ein Hohlraum verbleiben, der nachträglich mit dem Kern ausgefüllt werden kann.

Bei unterschiedlichen Querschnittsflächen der Schaftenden kann die Faserzahl unter Beachtung des Querschnitts des dünneren Schaftendes bestimmt werden. Dabei wird am anderen Schaftende ein gewisser Hohlraum entstehen, der mit dem Kern ausgefüllt wird. Das dünnere Schaftende wird in der Regel der untere, in den Femurknochen eingeführte Teil des Schaftes sein. In diesem Fall kann es auch zweckmäßig sein, die Faserzahl anhand des Querschnittes des oberen Schaftendes zu berechnen, und zwar derart, daß dieses Ende kompakt mit Fasern gefüllt wird. Das untere Ende müßte in diesem Fall mechanisch abgedünnt werden.

Als Materialien können für Prothesen bekannte Werkstoffe verwendet werden, wobei vorzugsweise Kohlenstoffasern mit einem bekannten biokompatiblen Matrixmaterial zur Optimierung der Steifigkeit zum Einsatz kommen sollten.

Für die Schalen werden Endlosfasern verwendet, die in dem fertigen Schaft parallel zur Außenkontur des Schaftes in Längsrichtung liegen und damit eine optimale Druckbeanspruchung gewährleisten. Der Füllkörper kann nach irgendeiner bekannten Faserverbundtechnik hergestellt werden. Nachdem dieser keine besonderen Lasten, bis auf Radialdrücke, zu tragen hat, wird der Kern vorzugsweise im Preßverfahren aus Kurzfasern hergestellt. Dafür werden vorzugsweise die gleichen Materalien wie für die Schalen verwendet.

Der Füllkörper läßt sich zur Aufnahme von medizinisch technischen Einrichtungen sowie von medizinischen Versorungen, z. B. Medikamente, nutzen, um Kontrollmöglichkeiten und Heilungsunterstützungen nach der Implantation der Prothese zu schaffen.

Die Erfindung erstreckt sich auf ein weiteres Verfahren, wonach der Kern nicht vorgefertigt, sondern in flüssiger bis zähflüssiger Form oder als aufschäumbare Masse in den Hohlraum eingeführt, der beim Zusammenlegen der vorgefertigten Schalen gebildet wird.

Durch die geometrisch unregelmäßige Kontur eines Schaftes bilden die zusammengesetzten, die Ummantelung darstellenden Schalen ebenfalls einen geometrisch unregelmäßigen Hohlraum.

Durch das Einbringen eines Werkstoffes in flüssiger- oder schaumiger Form in den Hohlraum, ist es möglich, diesen vollständig auszufüllen und das mit fertigungstechnisch sehr einfachen Maßnahmen. Damit wird das Verfahren fertigungstechnisch optimiert, indem die sorgfältige Konturgebung eines vorgefertigten Kernes entfällt und ein lückenloser Verbund zwischen Kern und Schalen möglich ist.

Dadurch, daß die Zahl der Fasern über sämtliche Querschnitte des Schaftes konstant bleibt, ergibt sich in der Regel zumindest an einem Schaftende ein mit dem inneren Hohlraum in Verbindung stehender Kanal. Dieser Kanal wird genutzt, um nach dem Zusammensetzen der Schalen den Werkstoff für den Kern einzuführen. Es ist selbstverständlich möglich, an irgendeiner anderen geeigneten Stelle eine oder mehrere Öffnungen zum Einführen des Kernmaterials zu schaffen.

Dem Werkstoff für den Kern können Kurzfasern zur Aufrechterhaltung der Festigkeit gegen Radialkräfte, Kontrastmittel für medizintechnische Zwecke u. ä. zugemischt werden.

Die Schalen für die Ummantelung werden im zusammengesetzten Zustand, vorzugsweise innerhalb einer Form, gehalten. In diesem Fall ist es zweckmäßig, den Werkstoff für den Kern mit einem leichten Überdruck einzuführen, um die Schalen geringfügig gegen die Formwandung zu drücken und damit eine optimale Ausrichtung der Fasern zu erreichen. Um beim Aushärtungsprozeß einem Schrumpfvorgang entgegenzuwirken, wird der Werkstoff für den Kern mit entsprechend höherem Überdruck eingeführt. Ferner kann das Auskleiden des Hohlraumes mit einer Folie in manchen Anwendungsfällen zweckmäßig sein.

Die Erfindung wird anhand von in der Zeichnung schematisch dargestellten Ausführungsbeispielen näher beschrieben. Es zeigen:
- Fig. 1 und 2: einen Längs- bzw. Querschnitt eines Hüftgelenk-Prothesenschaftes,
- Fig. 3 und 4: einen Querschnitt und eine Ansicht einer Teilkomponente des Schaftes,
- Fig. 5 und 6: je eine Form zur Herstellung der Teilkomponente des Schaftes und
- Fig. 7: ein weiteres Ausführungsbeispiel.

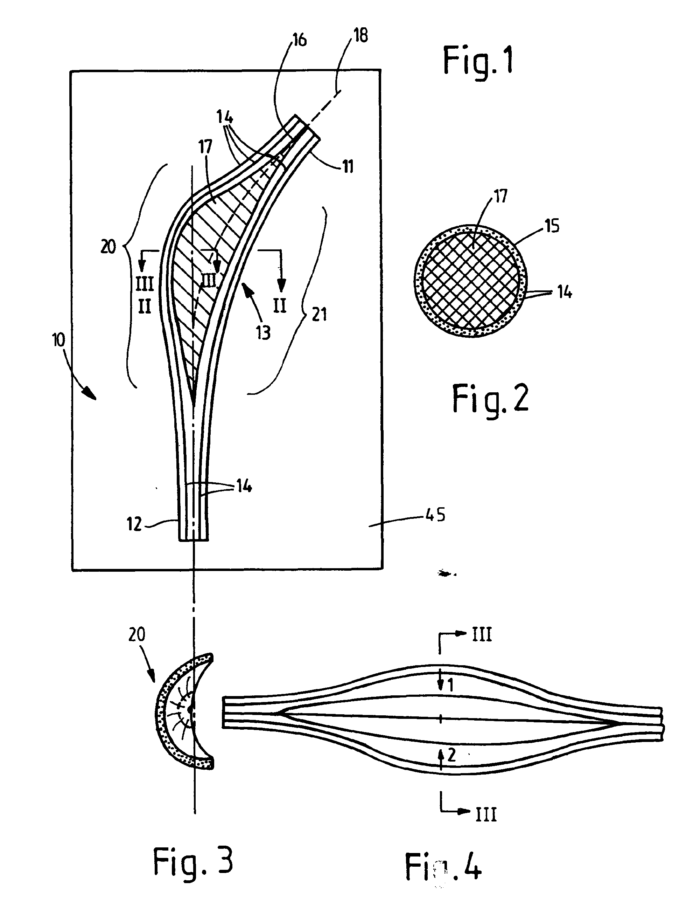

In Figur 1 ist ein Längsschnitt eines Schaftes 10 für Hüftgelenkprothesen dargestellt, der ein annähernd zylindrisches oberes Ende 11 für die Aufnahme eines nicht dargestellten Gelenkkopfes, ein unteres Ende 12 und einem im Querschnitt erweiterten, geknickten Mittelbereich 13 hat. Das untere Ende 12 ist vollständig aus längsgerichteten Fasern 14 gebildet, die über den mittleren Bereich 13 zum oberen Ende 11 führen. Aufgrund der Querschnittserweiterung im mittleren Bereich 13 und des größeren Querschnittes des oberen Endes 11 bilden die Fasern 14 im mittleren Bereich eine Außenhaut 15, während sie am oberen Ende 11 einen dünnen Kanal 16 freilassen. Der Hohlraum zwischen den Fasern 14 ist mit einem Kern 17 lückenlos ausgefüllt, der Radialkräfte aufzunehmen vermag.

In Figur 2, die einen Querschnitt durch den Mittelbereich 13 darstellt, ist die aus Fasern 14 gebildete Außenhaut 15 veranschaulicht, die den Kern 17 umgibt.

Die die Schaftenden 11 und 12 und die Außenhaut 15 bildenden Fasern 14 ummanteln vollständig den Kern 17, der lediglich eine Verdrängerfunktion hat. Die mechanischen Eigenschaften des Schaftes 10 werden im wesentlichen durch die längsgerichteten Fasern 14 bestimmt, deren Zahl über den gesamten Querschnitt des Schaftes 10 konstant bleibt. Daraus ergibt sich, daß die Querschnittsflächen, sowohl der Außenhaut 15 als auch der Schaftenden 11, 12, alle gleich sind. Dadurch ergibt sich eine optimale, vom oberen Schaftende 11 ausgehende Krafteinleitung in den Schaft 10 und das umliegende Knochenmaterial.

Zur Herstellung eines Schaftes gemäß Figur 1 werden zunächst Teilkomponenten des mit den Längsfasern 14 versehenen Bauteiles 11, 12, 13 hergestellt, wobei die Teilkomponenten durch Längsschnitte getrennte schalenartige Gebilde sind, die im folgenden Schalen genannt werden. Am einfachsten wird der Schaft aus zwei Schalenhälften gebildet, deren Fügefläche einem Längsschnitt des Schaftes 10 entspricht, dessen Schnittfläche senkrecht zur Biegefläche, d. h. senkrecht zur Zeichnungsfläche nach Figur 1 verläuft.

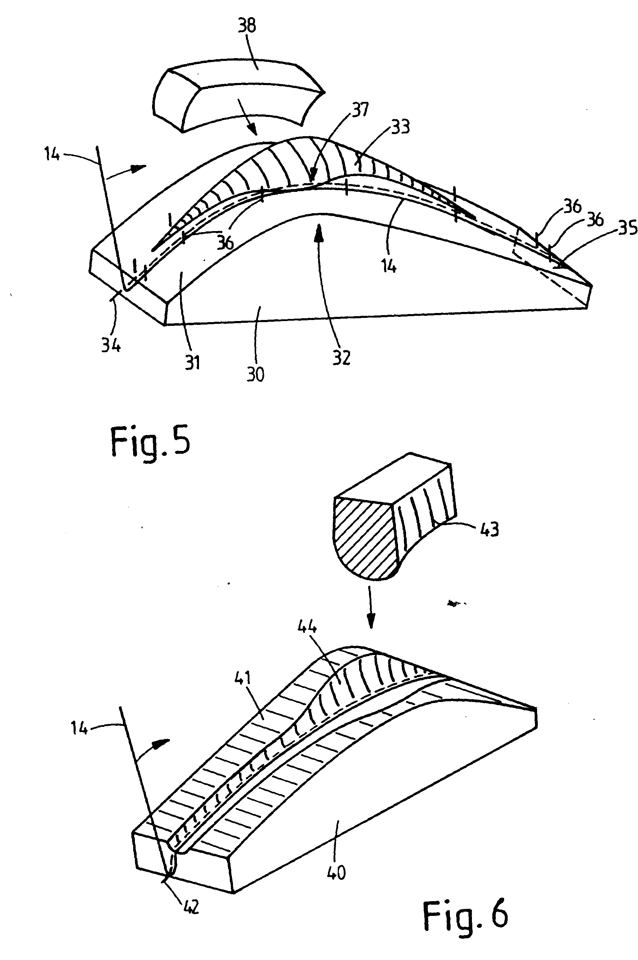

In Figur 3 ist ein Querschnitt entlang der Linien III - III der Figur 1 einer Schalenhälfte 20 gezeigt, deren Längsschnitt die linke Hälfte der Figur 1 ist. Eine Innenansicht dieser ersten Schalenhälfte 20 ist in Figur 4 gezeigt.

Zum Wickeln der ersten Schalenhälfte 20, die die konvexe Krümmung des Schaftes 10 einschließt, wird eine Form 30 gemäß Figur 5 verwendet, deren Wickelfläche 31 die Krümmung 32 des Schaftes 10 aufweist. Auf der Wickelfläche 31 bildet die Form 30 eine wulstartige Erhebung 33, die der Außenkontur der einen Hälfte des Mittelbereiches 13 unter Abzug der Wanddicke der Schale 15 entspricht. Der Wickelprozeß erfolgt durch Hin- und Herführen der Faser 14, wobei er an den Stirnseiten der Form 30 jeweils um einen Stift 34 bzw. 35 umgelenkt wird. Die durch eine nicht dargestellte Matrixtränkvorrichtung durchgezogene Faser 14 wird beispielsweise nach Figur 5 an der rechten Stirnseite befestigt und über die Wickelfläche 31 gezogen bzw. gelegt. Zur Führung der ersten Faser 14 sind Hilfsstifte 36 vorgesehen, die ein seitliches Wegrutschen der Fasern 14 verhindern.

Beim Erreichen der linken Stirnseite wird die Faser um den Umlenkstift 34 umgelenkt und, wie gestrichelt dargestellt, zur rechten Stirnseite geführt. Dabei legt sich die Faser im Anfangsbereich neben den ersten Faden und im Wulstbereich 37 auf den ersten Faden. Bei einem mechanischen Wickelprozeß wird die Form 30 beim Hin- und Herführen der Faser 14 pendelartig bewegt. Damit die sich übereinanderlegenden Fasern 14 im Wulstbereich 37 nicht abrutschen, wird eine Verdrängerform 38 verwendet, die unter Einhaltung eines Spaltes an den Wulst 33 angelegt wird.

In der Zeichnung ist der Einfachheit halber die Verdrängerform 38 für die in der Zeichnung dargestellte hintere Seite gezeichnet. Beim Wickeln der Zeichnungsvorderseite wird eine entsprechende Verdrängerform vorne angelegt. Wenn die Faser 14 den oberen Meridian erreicht, wird der Wickelprozeß von der hinteren unteren Seite fortgesetzt, so daß die Fasern sich im Wulstbereich 37 unter Zuhilfenahme der Verdrängerform 38 übereinanderlegen können. Die Reihenfolge und die Wickelrichtung ist in Figur 4 mit den Pfeilen 1 und 2 dargestellt.

In ähnlicher Weise wird die zweite Schalenhälfte 21 mit einer Form 40 gewickelt, deren konvex gewölbte Wickelfläche 41 dieses Mal nicht mit einem Wulst, sondern mit einer Vertiefung 44 versehen ist. Auch hier wird mit Hilfe von Umlenkstiften 42 die Faser 14 in Längsrichtung hin- und hergeführt, wobei sie in diesem Fall nicht von der Kante, sondern von der Mitte aus, d. h. von der tiefsten Stelle aus gewickelt wird, so daß auch hier die eine Faser sich auf die vorhergehend aufgebrachte Faser unter Zuhilfenahme einer Verdrängerform 43 auflegen kann. Nachdem die eine Hälfte der zweiten Schalenhälfte gewickelt ist, wird wieder von unten begonnen.

Es ist wichtig, daß die Faser 14 mit entsprechender Fadenspannung auf bzw. in die Form 30, 40 gelegt wird, so daß der Vorteil eines Wickelverfahrens für die mechanische Stabilität eines Formkörpers zum Tragen kommt.

Mit dem erfindungsgemäßen Verfahren ist die Faserzahl in jedem Querschnitt des Schaftes 10 die gleiche. Über die Faserdicke, den gewünschten Faseranteil und dem dünnsten Querschnitt des Schaftes wird die benötigte Faserzahl errechnet. Damit wird das eine Schaftende (beispielsweise Schaftende 12, Figur 1) voll mit gleichgerichteten Fasern 14 gefüllt sein, während das andere, etwas dickere Schaftende 11 einen entsprechenden zentralen Hohlraum bilden wird, wenn man die beiden Schalen 20, 21 aneinanderlegt. Im Mittelbereich 13 verteilen sich die Fasern homogen über die Außenhaut 15. Einer über den Schaftumfang ungleichmäßig verteilten Krafteinleitung kann damit Rechnung getragen werden, daß in dem Bereich mehr Fasern durchgeführt werden. Wenn z. B. nach Figur 1 die rechte Seite im Mittelbereich 13 steifer ausgebildet werden muß, dann wird man die Trennlinie 18 für die Schalen 20, 21 aus der zentrischen Lage derart verschieben, daß mehr Fasern in die rechte Schale 21 eingehen als in die linke Schale 20.

Damit zeigt sich, daß das erfindungsgemäße Verfahren eine große Flexibilität bietet, bei der sowohl die mechanische Stabilität als auch die Kontur des Schaftes so genau gewählt werden kann, wie es für einen optimalen Einsatz des Schaftes notwendig ist. Die genaue Formgebung läßt sich in einfacher Weise durch entsprechende Ausgestaltung der Wickelformen 30, 40 erzielen. Dabei ist es auch möglich, konische Schaftenden 11 oder 12 mit gleicher Stabilität zu bilden.

Der Kern 17 ist ein verdichteter Körper, dessen Außenkontur genau die Form des Hohlraumes hat, der beim Zusammenlegen der Schalen 20, 21 gebildet wird. Der Füllkörper kann unter Anwendung konventioneller Methoden aus irgendeinem Faserverbundwerkstoff hergestellt werden. Es werden vorzugsweise Kurzfasern verwendet und der Kern 17 im bekannten Preßverfahren her gestellt. Um etwaige Spannungen oder anderweitige Störungen zu vermeiden, wird für den Kern gleiches oder typenähnliches Material wie für die Schalen 20, 21 verwendet. Wenn für die Schalen 20, 21 beispielsweise Kohlenstoffasern verwendet werden, so können für den Kern 17 auch Kohlenstoffasern aber auch Pechfasern zur Anwendung kommen. Für die Matrix muß ein biokompatibles Material eingesetzt werden. Derartige Materialien sind aus der Literatur bekannt.

Der Kern läßt sich ebenfalls hohl ausbilden und, gefüllt mit Kontrastmitteln, Medikamenten und dergleichen, einsetzen.

Die Einzelkomponenten 17, 20, 21 werden nach ihrer Herstellung gemäß den vorherbeschriebenen Verfahren ungehärtet von den Formen genommen, zur Bildung des Schaftes 10 zusammengelegt und in eine Druckform 45 eingebracht.

Die Druckform 45 besteht aus zwei Formhälften mit Formmulden, die dem Schaft 10 mit geringem Untermaß entsprechen. Die Schnittfläche der Form 45 verläuft senkrecht zu der für die Herstellung der Schalen ideell gedachte Schnittfläche 18. In Figur 1 ist eine Formhälfte der Druckform 45 eingezeichnet. Nach dem Einlegen der entsprechend zusammengebrachten Teilkomponenten 17, 20 und 21 in die Druckform 45 werden die beiden Formhälften bis zum Anlegen der Formflächen zusammengedrückt, wobei der noch nicht gehärtete Schaft 10 unter entsprechendem Druck gehalten wird. Auf diese Weise werden der Schaft 10 bzw. die Teilkomponenten 17, 20, 21 bei entsprechender Temperatur ausgehärtet. Durch die passenden Formgebungen und den mit der Druckform 45 auferlegten Druck werden die Oberflächen der Teilkomponenten 17, 20, 21 derart kontaktiert, daß sie mehr oder weniger zusammenschmelzen, ohne Übergangsstellen zu hinterlassen.

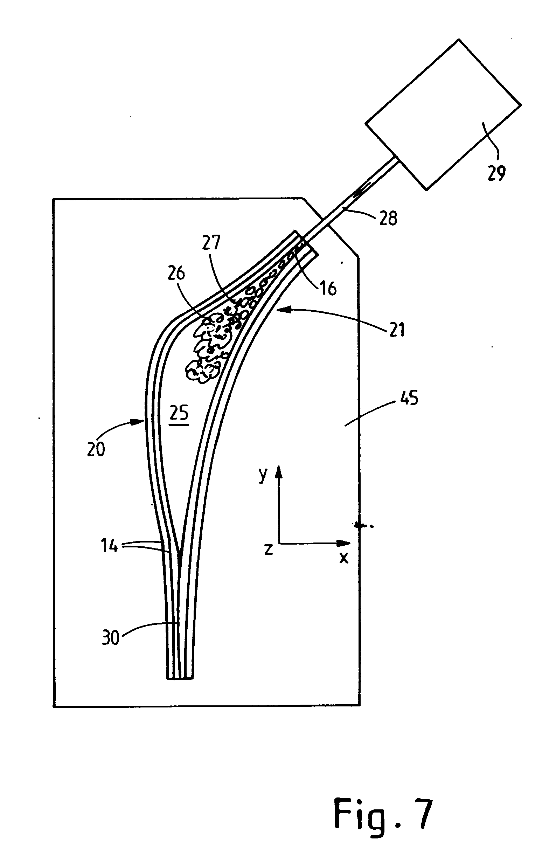

Fig. 7 zeigt ein Beispiel, bei dem nach der Herstellung der Schalen diese ungehärtet in eine zusammensetzbare Form eingelegt werden, von der eine Formhälfte 45 in der Zeichnung dargestellt ist. Die Kontaktfläche mit der zweiten Formhälfte liegt in einer XY-Ebene, während die Kontaktfläche bzw. Trennfläche 18 zwischen den Schalenhälften 20, 21 senkrecht zur YX-Ebene verläuft. In den zwei Formhälften 45, von denen nur eine in Figur 7 dargestellt ist, werden die Schalen 20, 21 zusammengehalten, wobei sie aufgrund ihrer Formgebung den Hohlraum 25 bilden, der über den Kanal 16 am oberen Schaftende von außen erreichbar ist. Durch den Kanal 16 wird der Werkstoff 26 über eine Zuführung 28 aus einem Reservoir 29 in den Hohlraum 25 gebracht. Der Werkstoff 26 kann eine aufschäumbare Masse, ein flüssiges Harz mit oder ohne Zusätze sein. Wichtig ist bei der Einführung des Werkstoffes 26 für den Kern 17, daß der Werkstoff den Hohlraum 25 möglichst vollständig ausfüllt. Die anzuwendende Methode richtet sich nach der Viskosität und den übrigen Eigenschaften des Werkstoffes 26. So ist es beispielsweise auch möglich, eine aufschäumbare Masse direkt aus einer Tube in den Hohlraum 25 hineinzudrücken. In der Regel wird jedoch eine Druckleitung 28 verwendet, über die der Werkstoff mit Überdruck gegenüber dem Atmosphärendruck einführbar ist. Dabei ist für eine ausreichende Entlüftung oder Evakuierung des Hohlraumes 25 zu sorgen. Als Einfüllstelle kann anstelle des Kanals 16 jede beliebig erzeugte Öffnung im Mittel- oder anderem Bereich des Schaftes verwendet werden.

Dem Werkstoff 26 können bei Bedarf Kurzfasern 27 sowie auch Kontrastmittel und dergleichen beigefügt werden.

Nach dem Füllen des Hohlraumes 25 mit einem geeigneten Werkstoff 26 wird der Schaft innerhalb der Formen 45 dem Härtungsprozeß unterzogen, wobei die Matrix der Schalen 20, 21 an den Fügestellen 30 in eine nahtlose Verbindung eingeht. In ähnlicher Weise erfolgt eine innige Verbindung zwischen der Matrix der Schalen 20, 21 mit dem Werkstoff 26. Ein Überdruck innerhalb des Hohlraumes 25 kann einen etwaigen Schrumpfprozeß entgegenwirken. Mit einer Auskleidung der Innenwandung der Schalen 20, 21 kann verhindert werden, daß ein innerer Überdruck die Faserstruktur der Schalen lokal beeinträchtigt.

## Patentansprüche

1. Schaft (10) für eine Hüftgelenkprothese aus faserverstärktem Kunststoff, wobei die Fasern (14) in Längsrichtung des Schaftes verlaufen und zumindest teilweise von einem Schaftende (11) zum anderen Schaftende (12) durchgehen, dadurch gekennzeichnet, daß der Schaft (10) aus einem Kern (17) und einer Ummantelung (20, 21) besteht, wobei die Ummantelung aus längsgerichteten Fasern (14) besteht, die alle von einem Schaftende (11) bis zum anderen Schaftende (12) durchgehen.

2. Schaft nach Anspruch 1, dadurch gekennzeichnet, daß die Querschnittsfläche der Ummantelung (20, 21) über die gesamte Länge des Schaftes annähernd konstant ist.

3. Verfahren zur Herstellung eines Schaftes (10) für Hüftgelenkprothesen aus faserverstärktem Kunststoff nach Anspruch ,1, bei dem mindestens zwei Schalen (20,21) des Schaftes mit entsprechenden Konturen je getrennt mit längs der Schaftachse (18) gerichteten und mit Matrix imprägnierten Endlosfasern (14) hergestellt werden, wobei die längsgerichteten Fasern von einem Schaftende (11) bis zum anderen Schaftende (12) durchgehen, und bei dem ferner ein Kern (17) hergestellt wird, dessen Außenkontur die Form des Hohlraumes (25) hat, der beim Zusammensetzen der Schalen gebildet wird, und wobei die Schalen um den Kern gelegt und alle Teilkörper (17,20,21) zur Bildung des Schaftes miteinander verbunden werden.

4. Verfahren nach Anspruch 3, dadurch gekennzeichnet, daß die Schalen (21, 22) und der Kern (17) vorgefertigt im nicht ausgehärteten Zustand zur Bildung des Schaftes (10) aneinandergelegt, gegebenenfalls mittels einer Form unter Druck zusammengehalten und schließlich unter Wärmeeinwirkung ausgehärtet werden.

5. Verfahren nach einem der Ansprüche 3 und 4, dadurch gekennzeichnet, daß zur Herstellung einer Schale (20,21) eine Form (30,40) verwendet wird, daß die Fasern (14) durch eine Imprägniereinrichtung geführt und in Pendelbewegung unter Verwendung von an der Form angebrachten Hilfsstiften (34, 35, 36, 42) sowie Verdrängerformen (38, 43) mechanisch auf bzw. in die Form (30, 40) gelegt werden, wobei die die Schaftenden (11, 12) bildenden Bereiche einer Schale nahezu mit unidirektionalen Fasern (14) gefüllt werden, daß mit den dadurch vorgegebenen Fasern der schalenartige Mittelbereich (13) gebildet wird, und daß anschließend die Fasern einer Gegenform mit geringem Druck gegen die Formwand angedrückt werden.

6. Verfahren nach Anspruch 5, dadurch gekennzeichnet, daß die eine Schale (20,. 21) durchlaufende Faserzahl von der Querschnittsfläche des unteren Schaftbereiches (12) bestimmt wird.

7. Verfahren zur Herstellung eines Schaftes für Hüftgelenkprothesen aus faserverstärktem Kunststoff nach Anspruch 1, wobei mittels Endlosfasern (14) mindestens zwei Schalen (20,21) des Schaftes mit entsprechenden Konturen hergestellt und zur Bildung des Schaftes miteinander verbunden werden, wobei jede Schale getrennt mit längs der Schaftachse gerichteten und mit Matrix imprägnierten Fasern (14) hergestellt wird und sämtliche Fasern von einem Schaftende bis zum anderen Schaftende durchgehen, wobei ferner ein Kern hergestellt wird, dessen Außenkontur die Form des Holhlraumes (25) hat, der beim Zusammensetzen der Schalen gebildet wird, und wobei der Werkstoff (26) für den Kern (17) nach dem Zusammensetzen der Schalen für die Ummantelung in den durch die Ummantelung gebildeten Hohlraum (25) eingebracht wird.

8. Verfahren nach Anspruch 7, dadurch gekennzeichnet, daß die vorgefertigten Schalen (20,21) im nicht ausgehärteten Zustand zusammengesetzt werden, daß eine aufschäumbare, flüssige oder zähflüssige Masse (26) in den Hohlraum (25) eingeführt und zur Bildung einer innigen Verbindung mit den Schalen ausgehärtet wird.

9. Verfahren nach Anspruch 7 oder 8, dadurch gekennzeichnet, daß der Werkstoff(26) für den Kern (17) Kurzfasern (27) enthält.

10. Verfahren nach einem der Ansprüche 7 bis 9, dadurch gekennzeichnet, daß der Werkstoff (26) für den Kern (17) mit Überdruck in den Hohlraum (25) der Ummantelung (20, 21) eingeführt wird.

11. Verfahren nach einem der Ansprüche 3 bis 10, dadurch gekennzeichnet, daß sämtliche Teilkomponenten (17, 20, 21, 26) eines Schaftes (10) aus gleichem Faser- und Matrixmaterial hergestellt werden.

## Claims

1. A stem (10) of a hip joint prosthesis made of fibre-reinforced plastics material, the fibres (14) extending in the longitudinal direction of the stem and at least partially from one stem end (11) to the other stem end (12), characterised in that the stem (10) comprises a core (17) and a casing (20, 21), the casing comprising longitudinally arranged fibres (14) which all extend from one stem end (11) to the other stem end (12).

2. A stem according to claim 1, characterised in that the cross sectional area of the casing (20, 21) is approximately constant over the entire length of the stem.

3. A method for manufacturing a stem (10) for hip joint prostheses made of fibre-reinforced plastics material according to claim 1, in which at least two shells (20, 21) of the stem are manufactured separately with corresponding contours and matrix-impregnated continuous fibres (14) arranged along the stem axis (18), the longitudinally arranged fibres extending from one stem end (11) to the other stem end (12), and in which a core (17) is also manufactured, whose external contour has the shape of the cavity (25) formed when the shells are placed together, and the shells are placed around the core and all component parts (17, 20, 21) are connected together to form the stem.

4. A method according to claim 3, characterised in that the shells (21, 22) and the core (17) are placed together in a prefabricated but unhardened state in order to form the stem (10), are optionally held together under pressure by means of a mould and are finally hardened by thermal action.

5. A method according to one of claims 3 and 4, characterised in that a mould (30, 40) is used for manufacturing a shell (20, 21), the fibres (14) are guided through an impregnating device and are mechanically placed onto or into the mould (30, 40) with a reciprocating movement with the aid of auxiliary pins (34, 35, 36, 42) which are fitted to the mould and displacement moulds (38, 43), the sections of a shell forming the stem ends (11, 12) being almost filled with unidirectional fibres (14), in that the shell-like central section (13) is formed with the fibres provided in this manner, and the fibres of a countermould are subsequently pressed with reduced pressure against the mould wall.

6. A method according to claim 5, characterised in that the number of fibres extending through a shell (20, 21) is determined by the cross sectional area of the lower stem section (12).

7. A method for manufacturing a stem for hip joint prostheses made of fibre-reinforced plastics material according to claim 1, at least two shells (20, 21) of the stem with corresponding contours being manufactured by means of continuous fibres (14) and being joined together in order to form the stem, each shell being manufactured separately with matrix-impregnated fibres (14) arranged along the stem axis and all fibres extending from one stem end to the other stem end, a core also being manufactured, whose outer contour has the shape of the cavity (25) formed when the shells are placed together, and the material (26) for the core (17) being introduced into the cavity (25) formed by the casing after the shells have been placed together to form the casing.

8. A method according to claim 7, characterised in that the prefabricated shells (20, 21) are placed together in an unhardened state, a foamable, fluid or viscous mass (26) is introduced into the cavity (25) and is hardened to form an intimate connection with the shells.

9. A method according to claim 7 or 8, characterised in that the material (26) for the core (17) comprises short fibres (27).

10. A method according to one of claims 7 to 9, characterised in that the material (26) for the core (17) is introduced with excess pressure into the cavity (25) of the casing (20, 21).

11. A method according to one of claims 3 to 10, characterised in that all components parts (17, 20, 21, 26) of a stem (10) are manufactured from the same fibre and matrix material.

## Revendications

1. Tige (10) de prothèse de hanche faite d'une matières plastique renforcée par des fibres (14) disposées selon la direction longitudinale de la tige et dont une partie au moins s'étend en continu d'une extrémité (11) à l'autre (12) de la tige, caractérisée en ce que la tige (10) est faite d'un noyau (17) enveloppé dans une gaine (20, 21) constituée de fibres (14) longitudinales allant d'une extrémité (11) de la tige à l'autre extrémité (12) de celle-ci.

2. Tige selon la revendication 1, caractérisée en ce que la surface de la section de la gaine (20, 21) est sensiblement constante sur toute la longueur de la tige.

3. Procédé de fabrication d'une tige (10) de prothèse de hanche faite d'une matière plastique renforcée par des fibres selon la revendication 1, dans lequel au moins deux coquilles (20, 21) constituant la tige avec des surfaces externes correspondantes, sont réalisées séparément au moyen de fibres continues (14) orientées selon l'axe longitudinal de la tige et imprégnées d'une matrice, les fibres longitudinales allant en continu d'une extrémité (11) à l'autre (12) de la tige, un noyau (17) étant également réalisé avec une surface externe présentant la forme du volume creux (25) que délimitent les coquilles par leur assemblage, de sorte que les coquilles entourent le noyau et que tous les composants (17, 20, 21) se trouvent réunis entre eux de manière à former la tige.

4. Procédé selon la revendication 3, caractérisé en ce que les coquilles (20, 21) et le noyau (17) préfabriqués et à l'état non durci, sont appliqués les uns contre les autres pour former la tige (10), le cas échéant maintenus en pression les uns sur les autres dans un moule, et enfin durcis par action de la chaleur.

5. Procédé selon l'une des revendications 3 et 4, caractérisé en ce qu'on utilise une forme (30, 40) pour réaliser une coquille (20, 21), on fait passer les fibres (14) dans un dispositif d'imprégnation, ces fibres sont déposées mécaniquement sur ou dans la forme, par un mouvement pendulaire et en utilisant des pointes auxiliaires (34, 35, 36, 42) portées par la forme, de sorte que les zones d'une coquille correspondant aux extrémités (11, 12) de la tige sont remplies par des fibres unidirectionnelles (14) qui réalisent en même temps la zone médiane (13) en forme de coquille, les fibres étant finalement appliquées sous une faible pression, contre la paroi de la forme par l'action d'une contre-forme.

6. Procédé selon la revendication 5, caractérisé en ce que le nombre de fibres constituant chaque coquille (20, 21) est déterminé en fonction de la surface de la section de la partie inférieure (12) de la tige.

7. Procédé de fabrication d'une tige de prothèse de hanche faite d'une matière plastique renforcée par des fibres selon la revendication 1, dans lequel au moins deux coquilles (20, 21) figurant la tige par leurs surfaces externes correspondantes, sont réalisées au moyen de fibres continues (14) et réunies ensuite pour former la tige, chaque coquille étant réalisée séparément au moyen de fibres orientées selon l'axe de la tige, imprégnées d'une matrice et s'étendant d'une extrémité à l'autre de la tige, un noyau ayant pour forme externe celle du volume creux (25) créé par l'assemblage des coquilles, étant également fabriqué par introduction d'un matériau (26) à l'intérieur du volume creux (25) situé à l'intérieur de la gaine formée par l'assemblage des coquilles.

8. Procédé selon la revendication 7, caractérisé en ce que les coquilles (20, 21) préfabriquées et à l'état non durci, sont assemblées, puis une masse liquide ou visqueuse (26) transformable en mousse, est introduite dans le volume creux (25) et enfin durcie de manière à réaliser une liaison interne avec les coquilles.

9. Procédé selon la revendication 7 ou 8, caractérisé en ce que le matériau (26) utilisé pour réaliser le noyau (17) contient des fibres courtes.

10. Procédé selon une des revendications 7 à 9, caractérisé en ce que le matériau (26) utilisé pour réaliser le noyau (17) est introduit sous pression dans le volume creux (26) à l'intérieur de la gaine (20, 21).

11. Procédé selon une des revendications 3 10, caractérisé en ce que tous les composants (17, 20, 21, 26) d'une tige (10) sont réalisés dans le même matériau, combinant fibres et matrice.
